(19) Europäisches Patentamt

European Patent Office

Office européen des brevets

(11) **EP 0 805 210 B1**

(12) **EUROPÄISCHE PATENTSCHRIFT**

(45) Veröffentlichungstag und Bekanntmachung des Hinweises auf die Patenterteilung:
**19.03.2003   Patentblatt 2003/12**

(51) Int Cl.$^7$: **C12P 7/60**

(21) Anmeldenummer: **97106678.2**

(22) Anmeldetag: **23.04.1997**

(54) **Verfahren zur Isolierung von 2-Keto-L Gulonsäure**

Process for the isolation of 2-keto-L-gulonic acid

Procédé d'isolement de l'acide 2-ceto-L-gulonique

(84) Benannte Vertragsstaaten:
**AT BE CH DE DK ES FR GB IT LI NL**

(30) Priorität: **30.04.1996  EP 96106829**
**20.02.1997  EP 97102766**

(43) Veröffentlichungstag der Anmeldung:
**05.11.1997   Patentblatt 1997/45**

(73) Patentinhaber: **F. HOFFMANN-LA ROCHE AG**
**4070 Basel (CH)**

(72) Erfinder:
• **Dümpelmann, Ralf**
**4334 Sisseln (CH)**

• **Keglevic, Tomislav**
**2252 Gumpoldskirchen (AT)**

(74) Vertreter: **Kellenberger, Marcus, Dr. et al**
**F.Hoffmann-La Roche AG**
**Patent Department (PLP),**
**124 Grenzacherstrasse**
**4070 Basel (CH)**

(56) Entgegenhaltungen:
**EP-A- 0 403 351          EP-A- 0 403 993**

• **DATABASE WPI Section Ch, Week 7728 Derwent Publications Ltd., London, GB; Class B05, AN 77-49650Y XP002103225 & JP 52 066684 A (SHIONOGI & CO LTD) , 2. Juni 1977**

**Beschreibung**

Die vorliegende Erfindung betrifft ein neues Verfahren zur Ueberführung eines in einer wässrigen Fermentationslösung gelösten Natriumsalzes der 2-Keto-L-gulonsäure in eine alkoholische Lösung der freien Säure durch Kristallisation des Salzes als Monohydrat aus der Fermentationslösung und anschliessende Protonierung zur 2-Keto-L-gulonsäure durch Reaktion des Salzes mit einer stärkeren Säure in alkoholischem Medium.

2-Keto-L-gulonsäure (KGA) ist bekanntlich ein wichtiges Ausgangsmaterial zur Herstellung von Ascorbinsäure (Vitamin C). Bei fermentativen Verfahren zur Herstellung von KGA wird die als Stoffwechselprodukt entstehende KGA durch Zugabe einer Base, z.B. Natrium- oder Calciumhydroxid, neutralisiert, um günstige Fermentationsbedingungen zu erhalten. Das Produkt der Fermentation ist eine wässrige, biomassehaltige Fermentationslösung, in der das KGA-Salz, d.h. Natrium- oder Calcium-2-keto-L-gulonat [NaKGA bzw. Ca(KGA)$_2$], gelöst vorliegt.

Zur technischen Herstellung der Ascorbinsäure bzw. des Ascorbates muss nun die fermentativ hergestellte KGA in ein organisches Lösungsmittel überführt werden. Mit Vorteil wird ein niederer Alkohol als Lösungsmittel verwendet. Durch Veresterung des Alkohols mit KGA und anschliessende Zugabe einer Base entsteht das Ascorbat in hoher Ausbeute. Alternativ dazu kann die KGA in einem organischen Lösungsmittel unter stark sauren Bedingungen zu Ascorbinsäure umgesetzt werden. Die Ausbeuten sind allerdings in der Regel etwas tiefer [siehe Helv. Chim. Acta <u>17</u>, 311-328(1934)].

Alle Verfahren zur Aufarbeitung der Fermentationslösung basieren auf drei Prozessschritten:

1. Protonierung des vorliegenden Natrium- oder Calcium-2-keto-Lgulonates zur freien Säure (z.B. NaKGA + H$^+$ → KGA + Na$^+$);

2. Entfernung des Wassers;

3. Entfernung der in der Fermentationslösung vorhandenen Biomasse, gelösten Proteine und anderen Verunreinigungen;

Kennzeichnend für das jeweilige Verfahren ist die Reihenfolge der Prozessschritte und deren spezifische Ausführung.

Die Protonierung (Schritt 1) kann in der wässrigen Fermentationslösung durch Zugabe von Säuren erfolgen. Gemäss der US-Patentschrift (USP) 3 381027 und der Europäischen Patentpublikation (EP) 359 645 erhält man die gelöste KGA durch Zugabe von Schwefelsäure zu Ca(KGA)$_2$ und Abtrennen des ausgefällten Calciumsulfats. Eine Protonierung kann ebenfalls durch Verwendung von Kationenaustauscherharzen erfolgen. Gemäss der EP 359 645 wird die wässrige Ca(KGA)$_2$-haltige Fermentationslösung durch einen Kationenaustauscher geleitet und die Calciumionen vollständig entfernt. Bei der Verwendung von Kationenaustauschern muss vorher die Biomasse vollständig entfernt werden (Schritt 3), z.B. mittels Mikrofiltration, um eine genügend lange Standzeit sicherzustellen.

Das Produkt der Protonierung ist eine wässrige, KGA-haltige Lösung mit einem pH-Wert, der deutlich unter 2,0 liegt (der pK$_S$-Wert von KGA ist 2,54). KGA wird hieraus durch Kristallisation, Extraktion (z.B. gemäss EP 359 042) oder Adsorption (z.B. gemäss der Chinesischen Patentpublikation 1097731A: siehe Chem. Abs. 124, 56570) isoliert und dadurch vom Wasser getrennt (Schritt 2). Die Kristallisation von KGA kann nur schwierig mit hohen Ausbeuten erfolgen, da die Löslichkeit mit z.B. 480 g/l bei 30°C sehr hoch ist. Extraktion und Adsorption sind technisch schwierige Verfahren, zumal Verunreinigungen aus der Fermentation vorhanden sind. Gemäss der EP 359 042 muss dementsprechend die Biomasse vor der Extraktion vollständig entfernt werden.

Gemäss der EP 403 993 kann auch isolierte, aber verunreinigte KGA zur Herstellung von Natriumascorbat verwendet werden. Verunreinigungen, wie Biomasse und Proteine, werden im weiteren Prozessverlauf durch Zugabe von Natrium- bzw. Kaliumbicarbonat und Fällung von nichtveresterter KGA eliminert.

Bei allen vorgenannten Verfahren erfolgt zuerst die Protonierung der KGA und dann die Entfernung des Wassers. Prinzipiell ist aber auch eine Umkehrung möglich, d.h. es wird zuerst das NaKGA oder Ca(KGA)$_2$ aus der Fermentationslösung isoliert, dabei Wasser entfernt (Schritt 2) und anschliessend die Protonierung durchgeführt (Schritt 1). So ist die Kristallisation von Natrium-2-keto-L-gulonat-Monohydrat (NaKGA·H$_2$O) gemäss den Japanischen Patentpublikationen (Kokai) 66684/1977 und 62894/1978 bekannt, und bei Löslichkeiten von z.B. 250 g/l bei 30°C sind deutlich höhere Ausbeuten zu erwarten, als bei der Kristallisation von KGA·H$_2$O. Beide Substanzen kristallisieren übrigens als Monohydrat, was meist nicht berücksichtigt wird. Die Schwierigkeit bei dieser Anordnung, d.h. Schritt 2 vor Schritt 1, liegt in der vollständigen Protonierung des NaKGA in organischen Lösungsmitteln, da NaKGA praktisch unlöslich ist.

Einige Versuche zur Protonierung von NaKGA in organischen Lösungsmitteln sind jedoch dokumentiert.

Gemäss der EP 91 134 kann kristallines NaKGA mit gasförmigem Chlorwasserstoff in einem Gemisch aus Ethanol und Aceton zu KGA umgesetzt werden. Das ebenfalls gebildete Natriumchlorid wird abgetrennt. Im weiteren Reaktionsverlauf findet unmittelbar unter den stark sauren Reaktionsbedingungen eine Umlagerung zu kristalliner Ascorbinsäure statt, wobei unerwünschte Nebenprodukte entstehen und die Ausbeuten deswegen mit 60-82% gering sind.

Gemäss der USP 5 391 770 kann NaKGA mit über 40%-igem Überschuss an konzentrierter Schwefelsäure in Methanol umgesetzt werden (Beispiel 10). Die Reaktionszeit inklusive Veresterung zum Methylester beträgt 4,5 Stunden bei 65°C und die Ausbeute 91,9%. Anschliessend wird der pH auf 4 erhöht und das Natriumsulfat abgetrennt. Bei diesen langen Reaktionszeiten und dem hohen Überschuss an Säure sind gemäss der EP 403 993 erhebliche Anteile an Zersetzungsprodukten zu erwarten.

EP 403 993 offenbart die Umsetzung eines Gemisches aus 50% KGA und 50% NaKGA mit einem etwa 50%-igen Überschuss an Schwefelsäure in Methanol (Beispiel 5). Nach einer Stunde Reaktionszeit unter Rückflussbedingungen wird filtriert. Die Ausbeute des erhaltenen Natriumsulfats entspricht etwa 60% der Theorie. Dementsprechend kann maximal 50% der KGA in Form des Natriumsalzes eingesetzt werden. Grundsätzlich sieht das Verfahren den Einsatz von KGA vor, wobei das erwähnte NaKGA in nachfolgenden Stufen entsteht und zurückgeführt wird.

Nachteilig bei den letzten beiden Verfahren sind der hohe verwendete Überschuss an Schwefelsäure und die langen Reaktionszeiten unter stark sauren Bedingungen. Bei stöchiometrischer Zugabe von Schwefelsäure und damit milderen Bedingungen bleibt üblicherweise ein erheblicher Teil des NaKGA im entstandenen Natriumsulfat eingeschlossen, und die Ausbeute sinkt entsprechend.

Gemäss der USP 5 391 770 ist auch bekannt, dass das Natriumsalz der Ascorbinsäure (NaASC) durch Zugabe von Schwefelsäure in Methanol zu Ascorbinsäure protoniert werden kann. Dabei liegt NaKGA als Verunreinigung von bis zu 9% vor. In Methanol als Lösungsmittel betragen die Ausbeuten der Ascorbinsäure zwischen 91% und 96% (Beispiele 12, 14 und 15). In einem Lösungsmittelgemisch von 75% Methanol und 25% Wasser ist die Ausbeute an Ascorbinsäure dagegen 99% (Beispiel 2). Offensichtlich sind die Ausbeuten in reinem Lösungsmittel beträchtlich niedriger als in dem Lösungsmittelgemisch, da ein bevorzugter Wassergehalt von 15-25% angegeben ist. Ursächlich ist die höhere Löslichkeit von NaASC im Gemisch gegenüber dem reinen Lösungsmittel, z.B. bei 40°C etwa 2 Gewichtsprozent (G%) gegenüber etwa 0,3 G%.

Bei niedrigen Löslichkeiten ist der Stofftransport erschwert, und die Reaktion eines schlecht löslichen Salzes mit Schwefelsäure zu dem sehr schlecht löslichen Natriumsulfat (Löslichkeit = 0,024 G% in Methanol) ergibt entsprechend schlechtere Ausbeuten. Probleme des Stofftransportes sind in verstärktem Mass für eine Reaktion von NaKGA·$H_2O$ zu erwarten, da die Löslichkeiten noch deutlich tiefer sind als diejenigen von Natriumascorbat (bei 40°C, < 0,01 G% in Methanol, <0,1G% in 90% Methanol/10% Wasser).

Der vorliegenden Erfindung liegt die Aufgabe zugrunde, ein Verfahren bereitzustellen, welches die Ueberführung des Natriumsalzes der 2-Keto-L-gulonsäure, das in einer wässrigen, verunreinigten Fermentationsbrühe vorliegt, auf möglichst einfache Art und Weise in die freie 2-Keto-L-gulonsäure in alkoholischer Lösung erlaubt, und zwar in hoher Ausbeute und mit grosser Reinheit. Dabei sollen die Nachteile der vorbekannten Verfahren, insbesondere die vollständige Entfernung von Biomasse, Proteinen usw., z.B. mittels Mikrofiltration, die Verwendung von Kationenaustauschern zur Entfernung der Metallionen aus den wässrigen Fermentationslösungen sowie die Kristallisation bzw. Trocknung von 2-Keto-L-gulonsäure, vermieden werden. Zudem sollen nur einfach zugängliche Chemikalien verwendet werden, und möglichst wenige Stufen nötig sein.

Im Rahmen der vorliegenden Erfindung wurde nun ein Verfahren gefunden, das die gestellten Anforderungen erfüllt und gemäss welchem das Natriumsalz der 2-Keto-L-gulonsäure aus einer, auch nur teilweise von Biomasse befreiten Fermentationslösung kristallisiert und das erhaltene Natrium-2-keto-L-gulonat-Monohydrat in eine alkoholische Lösung der freien 2-Keto-L-gulonsäure übergeführt wird. Die Protonierung zur 2-Keto-L-gulonsäure bzw. die Entfernung der Metallionen erfolgt hierbei ausschliesslich durch Reaktion in alkoholischem Medium. Es wurden nun ganz spezielle Reaktionsbedingungen gefunden, unter denen in alkoholischem Medium suspendiertes Natrium-2-keto-L-gulonat-Monohydrat (NaKGA-$H_2O$) in sehr guten Ausbeuten mit einer wasserarmen Säure zu der gelösten, freien 2-Keto-L-gulonsäure (KGA) und einem unlöslichen Salz reagiert. Erst dadurch wird die Kristallisation des NaKGA·$H_2O$ aus der wässrigen Fermentationslösung sinnvoll. Die bei bisherigen Methoden benötigten kritischen Schritte, wie das Ueberleiten der Fermentationslösung über einen Kationenaustauscher und Kristallisation bzw. Trocknen von KGA werden dadurch vermieden. Als Resultat des erfindungsgemässen Verfahrens liegt die KGA in alkoholischer Lösung vor, welche dann direkt in hohen Ausbeuten auf beliebige Art und Weise verestert und zu Ascorbat umgesetzt werden kann. Kennzeichnend für das erfindungsgemässe Verfahren ist demnach zum einen die Reihenfolge der Prozessschritte, d.h. zuerst wird NaKGA·$H_2O$ aus einer Fermentationsbrühe kristallisiert, d.h. das Wasser wird, abgesehen vom Kristallwasser, vollständig entfernt (Schritt 2, siehe oben), danach erfolgt die Protonierung (Schritt 1) vollständig in einem niederen Alkohol als Lösungsmittel durch Zugabe einer starken, wasserarmen Säure, z.B. Schwefelsäure. Es entsteht ein schwer lösliches Salz der Säure, z.B. Natriumsulfat, welches leicht abgetrennt werden kann. Zurück bleibt die gewünschte Lösung von KGA, welche wie oben erwähnt direkt verestert und zu Ascorbat umgesetzt werden kann. Ebenfalls kennzeichnend sind spezielle Reaktionsbedingungen der Protonierung, bei denen, trotz extrem niedrigen Löslichkeiten des eingesetzten NaKGA·$H_2O$ und des Produktes, z.B. Natriumsulfat, Ausbeuten von mehr als 97% realisiert werden können. Hierzu werden spezifische, bisher unbekannte Stoffeigenschaften des NaKGA·$H_2O$ ausge-

nutzt, wie weiter unten noch erläutert wird.

Gegenstand der Erfindung ist damit ein Verfahren zur Ueberführung des Natriumsalzes der 2-Keto-L-gulonsäure aus wässrigen Fermentationslösungen in eine alkoholische Lösung der freien Säure und gewünschtenfalls in den entsprechenden Ester der Säure, das dadurch gekennzeichnet ist, dass man

a) das Natrium-2-keto-L-gulonat-Monohydrat aus einer wässrigen Fermentationslösung durch Verdampfungs-, Kühlungs- oder Verdrängungskristallisation gewinnt und gewünschtenfalls das so erhaltene Kristallisat durch Mahlen zerkleinert,

b1) das in der Stufe a) gewonnene, gewünschtenfalls gemahlene Natrium-2-keto-L-gulonsäure-Monohydrat in einem niederen Alkohol, der Methanol, Ethanol, Propanol oder 1,2-Ethandiol ist, suspendiert, die Kristalle quellen lässt und danach eine wasserarme Säure, die jeweils konzentrierte Schwefelsäure, Salpetersäure, Salzsäure oder Phosphorsäure, oder gasförmiger Chlorwasserstoff ist, zugibt, wobei der gemessene pH-Wert über 1,5 liegen soll, oder

b2) das in der Stufe a) gewonnene, gewünschtenfalls gemahlene Natrium-2-keto-L-gulonat-Monohydrat zusammen mit einer etwa stöchiometrischen Menge einer oben erwähnten wasserarmen Säure unter Verwendung eines Nassmahlsystems in einen oben erwähnten niederen Alkohol gibt, wobei der gemessene pH-Wert über 1,5 liegen soll, oder

b3) eine verfahrenstechnische Kombination der Stufen b1) und b2) inklusive Rückführungen von Stoffströmen durchführt, und

c) das in der Stufe b1), b2) oder b3) gebildete Salz der zugegebenen Säure abtrennt und so eine alkoholische Lösung der 2-Keto-L-gulonsäure erhält, und gewünschtenfalls

d) die in der Stufe c) erhaltene alkoholische Lösung der 2-Keto-L-gulonsäure mit einer katalytischen Menge einer Säure oder mit einem sauren Kationenaustauscher behandelt, um die 2-Keto-L-gulonsäure mit dem Alkohol zu dem entsprechenden 2-Keto-L-gulonsäureester zu verestern.

Die vor dem eigentlichen erfindungsgemässen Verfahren erfolgte Fermentation liefert eine biomassehaltige, trübe Fermentationsbrühe. Die Kristallisation des in der Fermentationsbrühe vorliegenden Natriumsalzes der 2-Keto-L-gulonsäure [ Stufe a)] kann prinzipiell direkt aus dieser Brühe erfolgen; vorteilhaft erscheint aber eine vorgängige Abtrennung von ' mindestens 90% der Biomasse mittels Zentrifugation. Hierbei resultiert eine trübe, aber schlammfreie Fermentationslösung. Eine vollständige Entfernung von Biomasse und gelösten Proteinen vor der Kristallisation ist allerdings nicht notwendig.

Die Kristallisation des Natriumsalzes aus der wässrigen Fermentationsbrühe bzw. -lösung gemäss Verfahrensstufe a) erfolgt zweckmässigerweise in an sich bekannter Weise, indem man die Fermentationsbrühe bzw. -lösung einengt, die Lösung abkühlt oder ein weiteres Lösungsmittel zugibt, d.h. durch Verdampfungs-, Kühlungs- bzw. Verdrängungskristallisation. Hierbei sind die üblichen Randbedingungen zu beachten. So sollte zum Beispiel die Verdampfungskristallisation unter vermindertem Druck und damit bei geringer Temperatur, vorzugsweise bei Temperaturen im Bereich von etwa 35°C bis etwa 60°C, durchgeführt werden, um eine Zersetzung des Produktes möglichst zu vermeiden. Die Kristallisation kann kontinuierlich oder diskontinuierlich durchgeführt werden, vorzugsweise kontinuierlich. Als Kristallisationsmethode verwendet man vorzugsweise kontinuierliche Verdampfungskristallisation. Das Kristallisat kann anschliessend durch eine fest/flüssig-Trennoperation, wie Filtration oder Zentrifugation, von der Mutterlauge abgetrennt werden. Das erhaltene Kristallisat NaKGA·$H_2O$ ist unter den angegebenen Bedingungen in der Regel reiner als 98% mit geringen Anteilen an organischen Verunreinigungen (<500 ppm Stickstoff), welche aber anschliessend in vorteilhafter Weise eliminiert werden.

In der nächsten Verfahrensstufe [b)] wird das Kristallisat in kristalliner oder gegebenenfalls durch Mahlen verkleinerter Form zunächst in dem niederen Alkohol, d.h. in Methanol, Ethanol, Propanol oder 1,2-Ethandiol (Glykol), vorzugsweise Methanol, suspendiert. Es wurde nun gefunden, dass sich das NaKGA-$H_2O$ unter Abgabe des Wassers in das Anhydrat (NaKGA) umwandelt und dabei viele, sehr dünne Nadeln (< 2 μm) ausbildet. Diese Nadeln weisen eine wesentlich höhere Oberfläche auf als das eingesetzte Monohydrat, wodurch oberflächenabhängige Reaktionen begünstigt werden. Diese Art von Dehydratisierung unter Bildung von neuen, nadelförmigen Kristallen wurde bisher nur durch Temperaturerhöhung beobachtet [siehe diesbezüglich "Dehydratisierung von Methandriol" in M.Kuhnert-Brandstätter, Theromicroscopy of Organic Compounds, Wilsons and Wilson's Comprehensive Analytical Chemistry, G.Svehla (Ed.), Elsevier, Amsterdam, Vol. 16, S. 355 (1982)]. Unter Berücksichtigung dieser spezifischen Stoffeigenschaften werden folgende Reaktionen formuliert:

1. Dehydratisierung

$$2NaKGA \cdot H_2O(\text{ungelöst}) \rightarrow 2NaKGA \text{ (ungelöst, Nadeln)} + 2H_2O$$

2. Protonierung

$$2NaKGA \text{ (ungelöst, Nadeln)} + H_2SO_4 \rightarrow 2KGA(\text{gelöst}) +$$

$$Na_2SO_4 \text{ (ungelöst)}$$

Für die Protonierung wird eine starke, wasserarme Säure verwendet. Der Wassergehalt der zugegebenen "wasserarmen" Säure ist im Prinzip nicht kritisch für das Verfahren. Die Konzentration von Wasser in der resultierenden 2-Keto-L-gulonsäure - Alkohol - Lösung bestimmt aber den Gleichgewichtsumsatz einer nachfolgenden Veresterung. Aus technischwirtschaftlichen Gesichtspunkten werden deshalb vorzugsweise wasserarme Säuren, d.h. Säuren, die eher als konzentriert zu bezeichnen sind, verwendet. Als wasserarme Säuren kommen gewisse konzentrierte Mineralsäuren, und zwar jeweils konzentrierte Schwefelsäure, Salpetersäure, Salzsäure und Phosphorsäure, und sogar gasförmiger Chlorwasserstoff in Frage. Vorzugsweise verwendet man konzentrierte Schwefelsäure oder Salzsäure, insbesondere > 95%ige Schwefelsäure, wobei sich in der Stufe b) das Natriumsalz der entsprechenden Säure, z.B. Natriumsulfat, bildet, welches in alkoholischem Medium praktisch unlöslich ist und deshalb leicht abgetrennt werden kann. Die Säure wird vorzugsweise in stöchiometrischen Mengen oder einem geringfügigen Ueberschuss (im allgemeinen in einem kleiner als 5-prozentigen Ueberschuss) zugegeben.

Infolge der Suspension des Natriumsalzes in dem niederen Alkohol quellen die Kristalle des Salzes.

Kennzeichnend für das erfindungsgemässe Verfahren sind nun Reaktionsbedingungen, welche die Umsetzung entsprechend den Reaktionen 1. und 2. unterstützen. In bezug auf die drei Verfahrensvarianten b1), b2) und b3) gilt folgendes:

b1) Zum einen werden hohe Ausbeuten erzielt, wenn die NaKGA·H$_2$O-Kristalle zuerst im alkoholischen Lösungsmittel suspendiert wird und dabei quellen (Umwandlung des NaKGA·H$_2$O zu nadelförmigen, grosse Oberflächen aufweisenden Kristallformen). Die Nadeln bilden sich dabei innert Sekunden bis zu Stunden, je nach Partikelgrösse des eingesetzten Materials und der Rührintensität. Vorzugsweise wird entweder feines Material (<100 μm) eingesetzt oder/und ein Intensivrührer oder Dispergator verwendet. Die Nadeln bilden sich dann normalerweise in deutlich weniger als 10 Minuten. Vorzugsweise wird weniger als 10 G% NaKGA·H$_2$O bezogen auf das Lösungsmittel (niederer Alkohol) eingesetzt, da die Suspension aufgrund der Nadelbildung sehr schlecht rührbar ist. Höhere Konzentrationen lassen sich durch Wiederholungen der Reaktionen 1. und 2. oder andere Rückführungen realisieren. Im Anschluss an die Nadelbildung wird die wasserarme Säure zugeleitet, wobei der pH-Wert über 1,5 liegen soll, vorzugsweise zwischen 2,5 und 3,5. Tiefere pH-Werte ergeben mehr Einschlüsse des NaKGA im Salz, und es werden, besonders mit Schwefelsäure, vermehrt unerwünschte Nebenprodukte gebildet.

b2) Hohe Ausbeuten können auch bei gleichzeitiger Zugabe des NaKGA·H$_2$O und der Säure erzielt werden, aber nur, wenn Partikeln mittels Nassmahlung zerkleinert werden, wie z.B. mit Rotor-Stator-Dispergiermaschinen, Homogenisatoren, Ultraschall oder ähnlichen Geräten. Der pH-Wert soll auch hier oberhalb von 1,5, vorzugsweise 2,5-3,5, sein, damit die Reaktion nicht zu schnell abläuft und zumindest im mikroskopischen Massstab eine Nadelbildung stattfindet, welche die Umsetzung unterstützt. Die Reaktionszeit ist üblicherweise weniger als 20 Minuten; je nach Nassmahlung sind längere Zeiten nötig. Die durchschnittliche Partikelgrösse des entstandenen Salzes sollte maximal 10 μm betragen, vorzugsweise < 3 μm.

Ein Nachteil der Variante b2) ist der notwendige hohe Energieeintrag zur Nassmahlung. Ein Vorteil ist allerdings die einfache Reaktionsführung, insbesondere die Vermeidung einer schlecht rührbaren Suspension, wie in der Variante b1).

b3) Mit Vorteil werden Kombinationen der Varianten b1) und b2) eingesetzt. Zum Beispiel kann in nachfolgenden Reaktoren entweder die Nadelbildung oder die Säurezugabe erfolgen und in einem weiteren die Nassmahlung eingesetzt werden. Ferner kann in einem kontinuierlichen Verfahren in einer ersten Stufe die Nadelbildung [Variante b1)] und in der nachfolgenden Stufe eine Nassmahlung mit Zufuhr der Säure [Variante b2)] erfolgen. Weiterhin kann auch das schwerlösliche Salz zum Beispiel mittels Hydrocyclone selektiv zurückgeführt werden. Dadurch werden die Verweilzeiten des schwerlöslichen Salzes erhöht und die erzielbaren Ausbeuten nochmals gesteigert. Es kann auch ein Natriumsulfat, welches beträchtliche Anteile an NaKGA·H$_2$O enthält, in dem Lösungsmittel suspendiert werden. Es bilden sich die typischen Nadeln, und die Reaktion mit einer wasserarmen Säure kann gemäss Variante b1) mit hohen Ausbeuten realisiert werden.

Für alle drei Varianten b1) - b3) gibt es keine Temperatureinschränkung. Vorzugsweise liegen die Temperaturen allerdings im Bereich von etwa 20°C bis etwa 70°C. Bei tieferen Temperaturen ist weniger KGA löslich, bei höheren Temperaturen treten Zersetzungen auf. Allgemein gilt, dass die Reaktionsbedingungen so zu wählen sind, dass praktisch keine Veresterungsreaktionen stattfinden können. Eine allfällig erwünschte Veresterung der 2-Keto-L-gulonsäure [Stufe d)] kann problemlos nach dem Abtrennen des unlöslichen Salzes, z.B. des Natriumsulfates, in bekannter Weise in Gegenwart einer Säure als Katalysator erfolgen.

Zu beachten ist, dass pH-Angaben streng genommen nur für wässrige Lösungen definiert sind. Die hier angegebenen pH-Werte beruhen auf Messungen mit einer pH-Glaselektrode mit 3-molarer Kaliumchlorid-Lösung als Elektrolyt. Bei Verwendung von anderen Messinstrumenten können unter ansonsten identischen Bedingungen durchaus andere pH-Werte gemessen werden. Die Angabe eines pH-Bereiches ist deshalb problematisch. Unter den hier angegebenen pH-Werten >1,5 entstehen üblicherweise deutlich weniger als 5% Ester.

Die freigesetzte 2-Keto-L-gulonsäure sollte unter den gewählten Reaktionsbedingungen im Reaktionsmedium löslich sein.

Die Abtrennung des schwerlöslichen Salzes in der Verfahrensstufe c) sowie die fakultative Veresterung der 2-Keto-L-gulonsäure in der Stufe d) können jeweils in bekannter Weise durchgeführt werden. So kann die Abtrennung des Salzes durch Filtration und/oder Zentrifugation durchgeführt werden, vorzugsweise durch Zentrifugation. Es sind allerdings grundsätzlich alle Fest/Flüssig-Trennmethoden für Partikel <10 μm denkbar. Die erhaltene klare Lösung von KGA kann anschliessend durch Zugabe einer katalytischen Menge einer Säure oder durch Verwendung eines sauren Ionenaustauschers, z.B. gemäss der EP 671 405, zu dem entsprechenden Ester umgesetzt werden.

Mit Vorteil werden wasserfreie Lösungsmittel und wasserarme, konzentrierte Säuren eingesetzt, da Wasser das Gleichgewicht der nachfolgenden Veresterung in ungünstiger Weise verschiebt. Oft ist natürlich etwas Wasser anwesend, sei es durch konzentrierte 37%-ige Salzsäure oder rückgeführte Stoffströme. Vorzugsweise soll aber der Wassergehalt 10% nicht übersteigen.

Mit Hilfe des erfindungsgemässen Verfahrens kann die für die Herstellung von Vitamin C sehr bedeutsame 2-Keto-L-gulonsäure, welche in der wässrigen Fermentationslösung als gelöstes Salz anfällt, auf relativ einfache und wirtschaftliche Weise in die alkoholische Lösung der freien Säure übergeführt werden. Die so erhaltene Lösung von 2-Keto-L-gulonsäure weist eine hohe Reinheit auf und kann in bekannter Weise in Ascorbinsäure übergeführt werden.

Wesentlicher Vorteil des erfindungsgemässen Verfahrens besteht darin, dass in der ersten Stufe [a)] eine trübe, noch Biomasse enthaltende Fermentationslösung zur Kristallisation des Natrium-2-keto-L-gulonatmonohydrats in hoher Ausbeute, d.h. deutlich höher als 90%, verwendet werden kann. Durch die anschliessend in der Stufe b) erfolgende Reaktion mit einer wasserarmen Säure (Protonierung) wird ein schwer lösliches Salz gebildet, mit welchem Reste von Biomasse oder Proteinen zum grössten Teil abgetrennt werden können. Solchermassen kann in dieser Stufe b) eine klare Lösung von 2-Keto-L-gulonsäure im Alkohol in hoher Ausbeute erhalten werden, die praktisch keine Biomasse enthält. Die Schwierigkeit einer vollständigen Biomasse- oder Proteinabtrennung in der wässrigen Phase wird dadurch vermieden.

Die folgenden Beispiele zur Ueberführung des Natriumsalzes der 2-Keto-L-gulonsäure aus wässrigen Fermentationslösungen in die alkoholische Lösung der freien Säure zeigen vorteilhafte Ausführungsformen des erfindungsgemässen Verfahrens auf, sollen jedoch keinerlei Einschänkung darstellen. Alle Temperaturen sind in Celsiusgraden (°C) angegeben.

Beispiel 1

Das Natriumsalz von 2-Keto-L-gulonsäure (als Monohydrat; nachfolgend NaKGA·H$_2$O) wurde wie folgt aus der Fermentationsbrühe kristallisiert:

2195 g einer wässrigen, biomassehaltigen Fermentationsbrühe wurden während 10 Minuten bei 12000 g zentrifugiert, was 2160 g eines trüben Überstandes und 35 g Schlamm, entsprechend 1,5%, ergab. Der Überstand wurde unter vermindertem Druck bei 20° eingeengt, das anfallende erste Kristallisat filtriert und mit Wasser gewaschen. Das Waschwasser wurde zur verbleibenden Lösung zurückgeführt. Diese wurde erneut bei 20° eingeengt, das zweite Kristallisat abfiltriert und mit Wasser gewaschen. Rückführung des Waschwassers, Einengen, Filtrieren und Waschen wurden ebenso für das dritte und vierte Kristallisat durchgeführt.

Die Kristallisation von 2160 g zentrifugierter, trüber Fermentationsbrühe ergab

| | |
|---|---|
| 132,90 g | 1. Kristallisat mit 99,3% Reinheit (54 % Ausbeute) |
| 60,17 g | 2. Kristallisat mit >99,5% Reinheit (78,9 % Gesamtausbeute) |

(fortgesetzt)

| 29,70 g | 3. Kristallisat mit 96,6% Reinheit (90,6 % Gesamtausbeute) |
| 15,00g | 4. Kristallisat mit 79,5% Reinheit (95,5 % Gesamtausbeute) |

Die Gesamtausbeute der ersten drei Kristallisationen betrug 91% mit einer durchschnittlichen Reinheit von ca. 99%.

Beispiel 2

Eine zentrifugierte, aber trübe, vorkonzentrierte Fermentationsbrühe wurde in einem 6 1 Kristaller unter Vakuum bei 55° kontinuierlich kristallisiert. Im stationären Zustand wurde kontinuierlich 1550 g/h Fermentationslösung mit 17,0 G% Natrium-2-keto-L-gulonat (NaKGA) zugeführt, entsprechend 1,22 Mol. Im Durchschnitt fielen 241 g/h trockenes Kristallisat mit einem Gehalt an NaKGA·$H_2O$ von 99% (1,02 mol/h) an. Ebenfalls wurden 180 g/h Mutterlauge mit 11,9% NaKGA (1,02 mol/h) erhalten. In Spül- und Reinigungslösungen wurden insgesamt 0,08 mol/h NaKGA wiedergefunden. Der Stickstoffgehalt der Mutterlauge betrug 2 G%. Die Kristalle enthielten 180 ppm Stickstoff.

Beispiel 3

40,0 g NaKGA·$H_2O$ mit einem Gehalt von 99% (169 mmol) wurden unter Rühren bei Raumtemperatur in 400 g Methanol suspendiert. Es resultierte eine schlecht rührbare, dicke Suspension. Nach 20 Minuten gab man innerhalb von 5 Minuten 8,73 g einer 95%-igen Schwefelsäure (85 mmol) bei einem minimalen pH von 2,0 zu. Nach 10 Minuten wurden weitere 20,0 g (85 mmol) NaKGA·$H_2O$ zugegeben und 5 Minuten später 4,4 g der 95%-igen Schwefelsäure (42 mmol) und weitere 10 Minuten gerührt. Die Zugabe von 20 g NaKGA·$H_2O$ und 4,4 g der 95%-igen Schwefelsäure wurde nochmals wiederholt. Nach 75 Minuten Rühren bei Raumtemperatur wurde die Lösung filtriert. Es fielen 25,21 g Natriumsulfat mit einem Gehalt von 3,6% an 2-Keto-L-gulonsäure (KGA; 5 mmol) an. Das erhaltene Filtrat enthielt 329 mmol KGA und 3 mmol 2-Keto-L-gulonsäure-methylester (MeKGA), was einer Ausbeute von 98% entsprach. Zusammen mit der im Filterrückstand gefundenen KGA betrug die Wiederfindung nahezu 100%.

Beispiel 4

80,0 g NaKGA·$H_2O$ mit einem Gehalt von 99,0% (338 mmol) und 17,63 g einer 95%-igen Schwefelsäure (171 mmol) wurden innerhalb von 15 Minuten bei 20-35° und einem pH-Wert grösser als 2,5 zu 390 g Methanol dosiert. Zur Nassmahlung wurde ein Dispergator mit Rotor-Stator-System im Reaktionsgefäss betrieben. Nach 60 Minuten Dispergieren wurde die Suspension filtriert und der Filterrückstand mit wenig Methanol gewaschen. Der pH-Wert blieb während der Dosierung durch die kontrollierte Zugabe der Schwefelsäure konstant bei 2,5. Nach 75 Minuten wurde ein pH-Wert von 2,6 gemessen. Das erhaltene Filtrat enthielt 330 mmol KGA, entsprechend einer Ausbeute von 98%. Es wurden 25,45 g Filterrückstand (Natriumsulfat) mit einem Gehalt an KGA von 5,7% erhalten, entsprechend 7 mmol. Der bestimmbare Gehalt an MeKGA betrug 0,04% im Filtrat (1 mmol) und war im Filterrückstand nicht nachweisbar. Die Wiederfindung an KGA und MeKGA im Filtrat zusammen mit der im Filterrückstand gefundenen KGA betrug somit nahezu 100%.

Beispiel 5

75,0 g NaKGA·$H_2O$ mit einem Gehalt von 99% (317 mmol) und 16,38 g einer 95%-igen Schwefelsäure (159 mmol) wurden innerhalb von 6 Minuten bei 60° bei einem pH-Wert von 2,5 zu 375 g Methanol dosiert. Zur Nassmahlung wurde ein Dispergator mit Rotor-Stator-System im Reaktionsgefäss betrieben. Nach der Zugabe wurde weitere 10 Minuten dispergiert, anschliessend filtriert und der Filterrückstand mit etwa 10 ml Methanol gewaschen und unter vermindertem Druck getrocknet. Das erhaltene Filtrat enthielt 303 mmol KGA und 5 mmol MeKGA, entsprechend einer Ausbeute von 97%. Es wurden 24,14 g Filterrückstand (Natriumsulfat) mit einem Gehalt an KGA von 6,2% erhalten, entsprechend 8 mmol. Der bestimmbare Gehalt an MeKGA betrug 0,27% im Filtrat (0,5 mmol) und 0,04% im Filterrückstand. Die Wiederfindung an KGA und MeKGA im Filtrat zusammen mit der im Filterrückstand gefundenen KGA bzw. dem MeKGA betrug somit nahezu 100%.

Beispiel 6

80,0 g kristallines NaKGA·$H_2O$ (338 mmol), erhalten gemäss der in Beispiel 2 beschriebenen Vorschrift, und 14,00 g einer 96%-igen Schwefelsäure (178 mmol) wurden innerhalb von 4 Minuten bei 65° bei einem pH-Wert grösser als 2,5 zu 400 g Methanol dosiert. Weitere 4,14 g (41 mmol) Schwefelsäure, insgesamt 5%-iger stöchiometrischer Überschuss, wurden während 2 Minuten zugegeben, wobei der pH-Wert auf 2,0 sank. Zur Nassmahlung wurde ein Dispergator mit Rotor-Stator-System im Reaktionsgefäss betrieben. Nach 10 Minuten wurde filtriert und der Filterrückstand mit 40 ml Methanol gewaschen. Das Filtrat enthielt 314 mmol KGA und 12,1 mmol MeKGA. Es wurden 25,02 g getrockneter Filterrückstand (Natriumsulfat) mit 7,5 mmol KGA und 0,5 mmol MeKGA erhalten. Die Ausbeute betrug 96,4 % bei einer Wiederfindung von 98,8%.

Beispiel 7

80,0 g kristallines NaKGA·$H_2O$ (338 mmol), erhalten gemäss der in Beispiel 2 beschriebenen Vorschrift, und 15,40 g einer 96%-igen Schwefelsäure (151 mmol) wurden innerhalb von 4 Minuten bei 65° bei einem pH-Wert grösser als 2,5 zu 400 g 1,2-Ethandiol dosiert. Weitere 1,88 g der Schwefelsäure (41 mmol) wurden innert einer Minute zugegeben, wobei der pH-Wert auf 2,3 sank. Zur Nassmahlung wurde ein Dispergator mit Rotor-Stator-System im Reaktionsgefäss betrieben. Nach 10 Minuten wurde filtriert und der Filterrückstand mit wenig 1,2-Ethandiol gewaschen.Es wurden 19,84 g getrockneter Filterrückstand (Natriumsulfat) mit 2,3 G% KGA, entsprechend 2,4 mmol KGA, erhalten. Das Filtrat konnte aufgrund des hohen Siedepunktes des Lösungsmittels nicht zufriedenstellend analysiert werden.

Beispiel 8

80,0 g kristallines NaKGA·$H_2O$ (338 mmol) erhalten gemäss der in Beispiel 2 beschriebenen Vorschrift, und 9.92 g einer 96%igen Schwefelsäure (97 mmol) wurden innerhalb von 5 Minuten bei 65° und einem pH-Wert von 2,5 in 432 g Methanol mit 8%-igem Wassergehalt dosiert. Innert 3 Minuten wurden weitere 7,36 g Schwefelsäure (72 mmol) zugegeben, wobei der pH-Wert minimal 1,9, gegen Ende 2,3 betrug. Zur Nassmahlung wurde ein Dispergator mit Rotor-Stator-System im Reaktionsgefäss betrieben. Nach 10 Minuten wurde die Suspension filtriert und der Filterrückstand mit 20 ml Methanol gewaschen. Es wurden 23,43 g trockener Filterrückstand mit 5,7% KGA (6,9 mmol) und 0,2 % MeKGA (0,2 mmol) erhalten. Das Filtrat enthielt 325 mmol KGA und 3,5 mmol MeKGA.

Beispiel 9

10,0 g Natriumsulfat, welches insgesamt 17,0 mmol NaKGA·$H_2O$, NaKGA und KGA enthielt, entsprechend 39,7 %-iger KGA, wurde bei 20°C in 100 g Methanol suspendiert. Nach 20 Minuten hatte sich eine dicke Suspension gebildet. Es wurde 0,82 g einer 95%-igen Schwefelsäure (7,9 mmol) so langsam zugegeben, dass der pH-Wert stets über 2,0 lag. Nach einer Stunde Rühren betrug der pH-Wert 2,3. Die Suspension wurde filtriert und mit wenig Methanol gewaschen. Es ergaben sich 7,0 g Filterrückstand (Natriumsulfat) mit 0,12% KGA. Eindampfen des Filtrates unter Vakuum ergab 3,47 g mit 81,4 % KGA und 6,5 % MeKGA.

Beispiel 10

In zehn aufeinanderfolgenden Versuchen wurde jeweils 400 g Methanol in einem 1 l Reaktionsgefäss vorgelegt und Natriumsulfat aus dem jeweilig vorangegangenen Versuch suspendiert. Jeweils 80,0 g kristallines NaKGA·$H_2O$ (3,38 mol), erhalten gemäss der in Beispiel 2 beschriebenen Vorschrift, wurden während 5 Minuten bei 60° zugegeben und jeweils 17,5 g einer 95%-igen Schwefelsäure (169 mmol) innert 10 Minuten bei einem pH-Wert von etwa 2,5 zugegeben. Zur Nassmahlung wurde ein Dispergator mit Rotor-Stator-System im Reaktionsgefäss betrieben. Nach weiteren 5 Minuten Reaktionszeit wurde filtriert und der feuchte Filterrückstand (Natriumsulfat) im nachfolgenden Versuch eingesetzt. Beim letzten Versuch wurde der Filterrückstand mit 400 ml Methanol gewaschen und getrocknet. Aus dem letzten Versuch wurden 240 g eines getrockneten Filterückstandes (Natriumsulfat) mit 3,9 % KGA und 0,3 % MeKGA (0,05 mol) erhalten. Filtratlösung und Waschwasser, total 3570 g, enthielten 16,6 % KGA (3,06 mol), 1,49 % MeKGA (0,25 Mol) und 11 ppm Stickstoff. Im Natriumsulfat wurden 350 ppm Stickstoff bestimmt. Die mittlere Korngrösse des Natriumsulfats sank innerhalb der Versuchsserie von 10 μm (1.Versuch) auf 3,7 μm (10.Versuch).

**Patentansprüche**

**1.**   Verfahren zur Ueberführung des in einer wässrigen Fermentationslösung vorliegenden Natriumsalzes der 2-Keto-L-gulonsäure in eine alkoholische Lösung der freien Säure und gewünschtenfalls in den entsprechenden Ester der Säure, **dadurch gekennzeichnet, dass** man

a) das Natrium-2-keto-L-gulonat-Monohydrat aus der wässrigen Fermentationslösung durch Verdampfungs-, Kühlüngs- oder Verdrängungskristallisation gewinnt und gewünschtenfalls das so erhaltene Kristallisat durch Mahlen zerkleinert,

b1) das in der Stufe a) gewonnene, gewünschtenfalls gemahlene Natrium-2-keto-L-gulonat-Monohydrat in einem niederen Alkohol, der Methanol, Ethanol, Propanol oder 1,2-Ethandiol ist, suspendiert, die Kristalle quellen lässt und danach eine wasserarme Säure, die jeweils konzentrierte Schwefelsäure, Salpetersäure, Salzsäure oder Phosphosäure, oder gasförmiger Chlorwasserstoff ist, zugibt, wobei der gemessene pH-Wert über 1,5 liegen soll, oder

b2) das in der Stufe a) gewonnene, gewünschtenfalls gemahlene Natrium-2-keto-L-gulonat-Monohydrat zusammen mit einer etwa stöchiometrischen Menge einer oben erwähnten wasserarmen Säure unter Verwendung eines Nassmahlsystems in einen oben erwähnten niederen Alkohol gibt, wobei der gemessene pH-Wert über 1,5 liegen soll, oder

b3) eine verfahrenstechnische Kombination der Stufen b1) und b2) inklusive Rückführungen von Stoffströmen durchführt, und

c) das in der Stufe b1), b2) oder b3) gebildete Salz der zugegebenen Säure abtrennt und so eine alkoholische Lösung der 2-Keto-L-gulonsäure erhält, und gewünschtenfalls

d) die in der Stufe c) erhaltene alkoholische Lösung der 2-Keto-L-gulonsäure mit einer katalytischen Menge einer Säure oder mit einem sauren Kationenaustauscher behandelt, um die 2-Keto-L-gulonsäure mit dem Alkohol zu dem entsprechenden 2-Keto-L-gulonsäureester zu verestern.

2. Verfahren nach Anspruch 1, **dadurch gekennzeichnet, dass** als zweite Stufe entweder die Stufe b1) oder die Stufe b2) eingesetzt wird.

3. Verfahren nach Anspruch 1 oder 2, **dadurch gekennzeichnet, dass** man als Kristallisationsmethode in der Stufe a) kontinuierliche Verdampfungskristallisation verwendet.

4. Verfahren nach einem der Ansprüche 1 bis 3, **dadurch gekennzeichnet, dass** man als niederen Alkohol in der Stufe b) Methanol verwendet.

5. Verfahren nach einem der Ansprüche 1 bis 4, **dadurch gekennzeichnet, dass** man als wasserarme Säure in der Stufe b) konzentrierte Schwefelsäure oder Salzsäure, vorzugsweise > 95%ige Schwefelsäure, verwendet.

6. Verfahren nach einem der Ansprüche 1 bis 5, **dadurch gekennzeichnet, dass** in der Stufe b) der pH-Wert zwischen 2,5 und 3,5 liegt.

7. Verfahren nach einem der Ansprüche 1 bis 6, **dadurch gekennzeichnet, dass** die Stufe b) bei Temperaturen im Bereich von etwa 20°C bis etwa 70°C erfolgt.

8. Verfahren nach einem der Ansprüche 1 bis 7, **dadurch gekennzeichnet, dass** in der Stufe c) die Abtrennung des gebildeten Salzes durch Filtration und/oder Zentrifugation, vorzugsweise durch Zentrifugation, durchgeführt wird.

**Claims**

1. A process for the conversion of the sodium salt of 2-keto-L-gulonic acid, which is present in an aqueous fermentation solution, into an alcoholic solution of the free acid and, if desired, into the corresponding ester of the acid, which process comprises

a) recovering sodium 2-keto-L-gulonate monohydrate from an aqueous fermentation solution by evaporation, cooling or displacement crystallization and, if desired, pulverizing the thus-obtained crystallizate by grinding,

b1) suspending the optionally ground sodium 2-keto-L-gulonate monohydrate obtained in step a) in a lower alcohol, which is methanol, ethanol, propanol or 1, 2-ethanediol, leaving the crystals to swell and thereafter adding an acid of low water content, which is in each case concentrated sulphuric acid, nitric acid, hydrochloric acid or phosphoric acid, or gaseous hydrogen chloride, whereby the measured pH value should be above 1.5, or

b2) adding the optionally ground sodium 2-keto-L-gulonate monohydrate obtained in step a) together with an about stoichiometric amount of an above-mentioned acid of low water content to an above-mentioned lower alcohol using a wet grinding system, whereby the measured pH value should be above 1.5, or

b3) carrying out a combination of steps b1) and b2) including recycling of product streams, and

c) separating the salt of the added acid formed in step b1), b2) or b3) and thus obtaining an alcoholic solution of 2-keto-L-gulonic acid, and, if desired,

d) treating the alcoholic solution of 2-keto-L-gulonic acid obtained in step c) with a catalytic amount of an acid or with an acidic cation exchanger in order to esterify the 2-keto-L-gulonic acid with the alcohol to give the corresponding 2-keto-L-gulonate.

2. A process according to claim 1, wherein either step b1) or step b2) is used as the second step.

3. A process according to claim 1 or 2, wherein continuous evaporation crystallization is used as the crystallization method in step a).

4. A process according to any one of claims 1 to 3, wherein methanol is used as the lower alcohol in step b).

5. A process according to any one of claims 1 to 4, wherein concentrated sulphuric acid or hydrochloric acid, preferably > 95% sulphuric acid, is used as the acid of low water content in step b).

6. A process according to any one of claims 1 to 5, wherein the pH value is between 2.5 and 3.5 in step b).

7. A process according to any one of claims 1 to 6, wherein step b) is carried out at temperatures in the range of about 20°C to about 70°C.

8. A process according to any one of claims 1 to 7, wherein in step c) the separation of the salt formed is carried out by filtration and/or centrifugation, preferably by centrifugation.

**Revendications**

1. Procédé pour la conversion du sel sodique de l'acide 2-céto-L-gulonique présent dans une solution aqueuse de fermentation, en une solution alcoolique de l'acide libre et si on le désire en l'ester correspondant de l'acide, **caractérisé en ce que**

a) on obtient le 2-céto-L-gulonate de sodium monohydraté à partir de la solution aqueuse de fermentation par cristallisation par évaporation, refroidissement ou déplacement et si on le désire on fragmente par broyage le produit de cristallisation ainsi obtenu,
b1) on met le 2-céto-L-gulonate de sodium monohydraté obtenu dans l'étape a), éventuellement broyé, en suspension dans un alcool inférieur qui est le méthanol, l'éthanol, le propanol ou le 1,2-éthanediol, on fait gonfler les cristaux et on ajoute ensuite un acide pauvre en eau, qui est l'acide sulfurique, nitrique, chlorhydrique ou phosphorique, chacun concentré, ou du gaz chlorhydrique, le pH mesuré devant être supérieur à 1,5, ou
b2) on introduit dans un alcool inférieur précité le 2-céto-L-gulonate de sodium monohydraté obtenu dans l'étape a), éventuellement broyé, avec une quantité approximativement stoechiométrique d'un acide pauvre en eau précité, en utilisant un système de broyage par voie humide, le pH mesuré devant être supérieur à 1,5, ou
b3) on effectue une combinaison des processus des étapes b1) et b2), y compris des recyclages de courants de substances, et
c) on sépare le sel de l'acide ajouté, formé dans l'étape b1), b2) ou b3), et on obtient ainsi une solution alcoolique de l'acide 2-céto-L-gulonique, et si on le désire

d) on traite la solution alcoolique de l'acide 2-céto-L-gulonique, obtenue dans l'étape c), par une quantité catalytique d'un acide ou par un échangeur de cations acide, pour estérifier avec l'alcool l'acide 2-céto-L-gulonique en l'ester d'acide 2-céto-L-gulonique correspondant.

2. Procédé selon la revendication 1, **caractérisé en ce qu'**on utilise en tant que deuxième étape soit l'étape b1), soit l'étape b2).

3. Procédé selon la revendication 1 ou 2, **caractérisé en ce qu'**on utilise en tant qu'étape de cristallisation dans l'étape a) une cristallisation par évaporation continue.

4. Procédé selon l'une quelconque des revendications 1 à 3, **caractérisé en ce qu'**on utilise comme alcool inférieur dans l'étape b) le méthanol.

5. Procédé selon l'une quelconque des revendications 1 à 4, **caractérisé en ce qu'**on utilise comme acide pauvre en eau dans l'étape b) de l'acide sulfurique ou chlorhydrique concentré, de préférence de l'acide sulfurique à > 95 %.

6. Procédé selon l'une quelconque des revendications 1 à 5, **caractérisé en ce que** dans l'étape b) le pH est compris entre 2,5 et 3,5.

7. Procédé selon l'une quelconque des revendications 1 à 6, **caractérisé en ce que** l'étape b) s'effectue à des températures dans la plage allant d'environ 20°C à environ 70°C.

8. Procédé selon l'une quelconque des revendications 1 à 7, **caractérisé en ce que** dans l'étape c) la séparation du sel formé s'effectue par filtration et/ou centrifugation, de préférence par filtration.